# EUROPÄISCHE PATENTSCHRIFT

(11) **EP 4 041 358 B1**
(45) Veröffentlichungstag und Bekanntmachung des Hinweises auf die Patenterteilung: **10.04.2024**
(21) Anmeldenummer: 20789923.8
(22) Anmeldetag: 08.10.2020
(51) Int. Cl.: A61B 5/087, A61M 16/10, G01K 11/12, G01K 13/024, A61M 16/08, A61M 16/00, A61B 5/08

(54) **KONNEKTOR FÜR EIN PATIENTEN-BEATMUNGSSYSTEM**
CONNECTOR FOR A PATIENT VENTILATION SYSTEM
RACCORD DESTINÉ À UN SYSTÈME DE VENTILATION DE PATIENT

(30) Priorität: 09.10.2019 DE 102019215483; 25.10.2019 DE 102019216487
(43) Veröffentlichungstag der Anmeldung: 17.08.2022
(73) Patentinhaber: Raumedic AG, 95213 Münchberg (DE)
(72) Erfinder: RUHLAND, Thomas, 95444 Bayreuth (DE); EHRENPFORDT, Ricardo, 08056 Zwickau (DE); SEUFERLING, Stefan, 91161 Hilpoltstein (DE)
(74) Vertreter: Schmitt-Nilson Schraud Waibel Wohlfrom Patentanwälte Partnerschaft mbB
(86) Internationale Anmeldenummer: PCT/EP2020/078220
(87) Internationale Veröffentlichungsnummer: WO 2021/069551

(56) Entgegenhaltungen:
- WO-A1-2010/139014
- JP-A- 2012 176 208
- US-A1- 2008 085 210

## Beschreibung

Die vorliegende Patentanmeldung nimmt die Prioritäten der deutschen Patentanmeldungen DE 10 2019 215 483.2 und DE 10 2019 216 487.0 in Anspruch, deren Inhalt durch Bezugnahme hierin aufgenommen wird.

Die Erfindung betrifft einen Konnektor für ein Patienten-Beatmungssystem.

Ein derartiger Konnektor ist bekannt aus der DE 20 2014 103 998 U1. Die US 2012/0125333 A1 offenbart ein Beatmungssystem. Die GB 2572468 A offenbart ein Sicherheitswarnsystem, welches bei einem Beatmungskreislauf zum Einsatz kommen kann. Die US 2012/0136267 A1 offenbart eine Vorrichtung und ein Verfahren zur sichtbaren Anzeige, ob eine Beatmung stattfindet. Die JP 2012-176 208 A offenbart Komponenten eines Beatmungssystems. Die WO 2010/139 014 A1 offenbart eine Nasenmaske für ein Beatmungssystem. Die US 2008/0085 210 A1 offenbart ein Verfahren zur Dekontamination von Beatmungs-Filtrationsmedien

Es ist eine Aufgabe der vorliegenden Erfindung, einen hinsichtlich seiner Einsatzmöglichkeiten flexiblen Konnektor für ein Patienten-Beatmungssystem derart weiterzubilden, dass eine Temperatur des beheizbaren Atemluft-Schlauchabschnitts zuverlässig angezeigt wird.

Diese Aufgabe ist erfindungsgemäß gelöst durch einen Konnektor mit den im Anspruch 1 angegebenen Merkmalen.

Erfindungsgemäß wurde erkannt, dass der Einsatz eines thermochromen Materials zur einfachen Möglichkeit führt, die Temperatur des entsprechenden Konnektorabschnitts und hierüber die Temperatur der geführten Atemluft anzuzeigen. Eine zusätzliche Anzeige kann entfallen. Auch aufwendige elektrische Konnektierungen oder elektrische Bauteile sowie eine entsprechende hiermit kommunizierende Sensorik können entfallen.

Bei dem Konnektor kann es sich in der einfachsten Ausführung um einen Atemluft-Schlauchabschnitt selbst handeln.

Das thermochrome Material kann eine Kunststoffmatrix aufweisen. Diese Kunststoffmatrix kann ein thermoplastisches Elastomer sein.

Die Kunststoffmatrix des thermochromen Materials kann ein Poly(organo)siloxan aufweisen. Es kann also ein thermochromes Material auf Silikonbasis zum Einsatz kommen. Hierdurch ist eine gute Bioverträglichkeit des thermochromen Materials gewährleistet.

Die Kunststoffmatrix des thermochromen Materials kann insgesamt aus Silikon sein. Es kann eine gesamte Komponente des Konnektors oder ggf. auch der gesamte Konnektor aus thermochromen Material ausgeführt sein. Alternativ kann das thermochrome Material beispielsweise als Streifen in den Konnektor eingesetzt sein.

Auch andere Materialien bzw. Materialzusammensetzungen, die in Verbindung mit thermochromem Material bekannt sind, können zum Einsatz kommen.

Mehrere Abschnitte des Konnektors sind aus jeweils einem thermochromen Material mit unterschiedlichen Farbumschlags-Temperaturen ausgeführt. Eine solche Ausführung des Konnektors ermöglicht es, nicht nur anzuzeigen, ob eine bestimmte Farbumschlags-Temperatur erreicht oder überschritten ist, sondern es wird innerhalb vorgebbarer Fehlergrenzen auch eine absolute Temperaturanzeige ermöglicht. Aufgrund der Mehrfachstruktur, die über die mehreren Abschnitte des Konnektors aus thermochromem Material mit unterschiedlicher Farbumschlags-Temperatur realisiert ist, können mehrere Temperaturschwellen überwacht werden. Es kann ein Temperaturbereich überwacht werden. Eine Anzahl der Abschnitte des Konnektors, die jeweils aus einem thermochromen Material mit unterschiedlicher Farbumschlags-Temperatur ausgeführt sind, kann eine Temperaturauflösung vorgeben. Beispielsweise kann ein Temperaturbereich zwischen 38°C und 42°C mit fünf Abschnitten überwacht werden, deren Farbumschlags-Temperatur sich jeweils um 1 K unterscheidet.

Hierüber kann zusätzlich zu den Abschnitten mit den unterschiedlichen Farbumschlags-Temperaturen auch eine Temperaturskala auf dem Konnektor aufgebracht sein. Auch eine Legende, die den verschiedenen Farben Temperaturen oder auch Warenhinweise zuordnen, kann auf den Konnektor direkt aufgebracht sein.

Der Konnektor hat mehrere Abschnitte, die jeweils aus einem thermochromen Material mit unterschiedlichen Farbumschlags-Temperaturen ausgeführt sind. Mindestens einer der Abschnitte ist dabei aus einem reversiblen thermochromen Material und mindestens ein weiterer der Abschnitte ist aus einem nicht reversiblen, thermochromen Material ausgeführt. Eine derartige Gestaltung des Konnektors gewährleistet beispielweise, eine bestimmte Temperaturüberschreitung und/oder eine bestimmte Temperaturunterschreitung dauerhaft festzuhalten. Eine Überschreitung eines Temperatur-Schwellwertes kann durch den Abschnitt aus nicht reversiblem thermochromem Material gespeichert werden. Ein zusätzlich möglicher Abschnitt des Konnektors aus einem reversiblen thermochromen Material kann genutzt werden, um einen aktuellen Temperaturwert des Konnektors zu überwachen.

Bei einem kontinuierlichen Farbverlauf des reversiblen/nicht reversiblen Abschnitts des Konnektors aus thermochromem Material in Abhängigkeit von der Temperatur kann die jeweilige Konnektortemperatur anhand einer Farbskala abgelesen werden.

Dies ermöglicht eine Beurteilung, ob über einen längeren Zeitraum ein bestimmter Betriebszustand, z. B. ein Fieberanfall des Patienten oder ein Ausfall eines Heizaggregats, stattgefunden hat.

Die Vorteile einer Baugruppe nach Anspruch 4 entsprechen denen, die vorstehend unter Bezugnahme auf den Konnektor bereits erläutert wurden.

Bei einer Baugruppe nach Anspruch 5 kann zudem die Temperatur der im Schlauchabschnitt geführten Atemluft über das thermochrome Material thermisch überwacht werden. Für die möglichen Gestaltungen des thermochromen Materialabschnitts gilt, was vorstehend zum entsprechenden thermochromen Materialabschnitt des Konnektors ausgeführt wurde. Grundsätzlich kann bei einer derartigen Baugruppe der Konnektor selbst auch ohne einen Abschnitt aus thermochromem Material ausgeführt sein.

Die vorstehend diskutierten Merkmale der Konnektoren der verschiedenen Lösungsaspekte können in beliebiger Form miteinander kombiniert vorliegen.

Ausführungsbeispiele der Erfindung werden nachfolgend anhand der Zeichnung näher erläutert. In dieser zeigen:
- Fig. 1: schematisch Hauptkomponenten eines Patienten-Beatmungssystems einschließlich eines Hauptaggregates, einer Befeuchtungseinrichtung, einer Atemmaske sowie mehrerer diese verbindende, die Atemluft führende, beheizbare Schlauchkomponenten einschließlich mehrerer Konnektoren zur fluidführenden Verbindung dieser Komponenten;
- Fig. 2: perspektivisch einen Endabschnitt einer der Schlauchkomponenten mit einem Anschluss-Konnektor und einem hiervon beabstandet, also noch nicht in Verbindungsposition, gezeigten Überwachungs-Konnektor,
- Fig. 3: eine weitere perspektivische Ansicht des Überwachungs-Konnektors;
- Fig. 4: eine Explosionsdarstellung, bei der eine wiederverwendbare Komponente des Überwachungs-Konnektors getrennt von einer Einweg-Komponente des Überwachungs-Konnektors dargestellt ist;
- Fig. 5: eine weitere Explosionsdarstellung des Überwachungs-Konnektors, wobei zusätzlich eine Platine der Einweg-Komponente als Bestandteil einer Startsignal-Generatoreinheit sowie eine Abdeckung hierfür dargestellt sind, welche zur Erzeugung eines Startsignals zur Erfassung einer Nutzungsdauer der Einweg-Komponente dienen;
- Fig. 6: einen teilweise innere Details preisgebenden Axialschnitt durch den Überwachungs-Konnektor, wobei insbesondere Komponenten einer Steuer/Regeleinheit, einer Lichtquelle und einer Startsignal-Generatoreinheit gezeigt sind;
- Fig. 7: perspektivisch in einer zu Fig. 2 ähnlichen Darstellung eine weitere Ausführung eines Überwachungs-Konnektors, der gleichzeitig die Funktion einer Anschluss-Konnektors vergleichbar zur Ausführung nach Fig. 2 aufweist;
- Fig. 8: eine weitere Ausführung einer Einweg-Komponente des Überwachungs-Konnektors mit einem Konnektorabschnitt aus einem thermochromen Material; und
- Fig. 9: abschnittsweise eine Ausführung einer Schlauchkomponente des Beatmungssystems mit einem Wandabschnitt aus einem thermochromen Material.

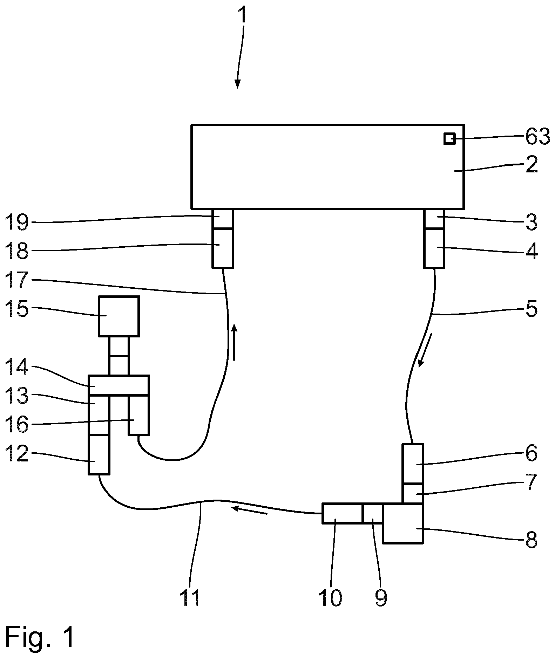

Ein Patienten-Beatmungssystem 1, dessen Hauptkomponenten in der Fig. 1 dargestellt sind, dient zur Beatmung eines Patienten im klinischen, sonstigen stationären oder auch häuslichen Pflegebereich.

Die wesentlichen, luftführenden Komponenten des Beatmungssystems 1 sind aus Kunststoff.

Das Beatmungssystem hat ein Hauptaggregat 2 zur Steuerung/Regelung insbesondere einer Atemluft-Zufuhr einer Luftbefeuchtung und einer Temperierung der Atemluft. Grundsätzlich kann das Hauptaggregat 2 auch zur Vorgabe einer Atemluft-Zusammensetzung dienen. Das Hauptaggregat 2 dient als Atemluftquelle.

Über einen Anschlussport 3 und einen Anschluss-Konnektor 4 steht das Hauptaggregat 2 mit einer Atemluft-Zuführ-Schlauchkomponente 5 in Fluidverbindung. Letztere ist wie auch die weiteren Schlauchkomponenten in der Fig. 1 sehr schematisch dargestellt. Die Schlauchkomponenten des Beatmungssystems 1 sind, wie nachfolgend noch beschrieben wird, gesteuert/geregelt über das Hauptaggregat 2 beheizbar.

Über einen weiteren Anschluss-Konnektor 6 steht die Atemluft-Zuführ-Schlauchkomponente 5 mit einem Anschlussport 7 einer Atemluft-Befeuchtungseinrichtung 8 in Fluidverbindung. Die Befeuchtungseinrichtung 8 steht in nicht dargestellter Weise mit dem Hauptaggregat 2 in Signalverbindung.

Über einen weiteren Anschlussport 9 und einen weiteren Anschluss-Konnektor 10 steht die Befeuchtungseinrichtung 8 mit einer Atemluft-Verbindungs-Schlauchkomponente 11 in Fluidverbindung.

Über einen weiteren Anschluss-Konnektor 12, einen Überwachungs-Konnektor 13 und einen Dreiwege-Konnektor 14 steht die Atemluft-Verbindungs-Schlauchkomponente 11 mit einer Patienten-Atemmaske 15 in Fluidverbindung. Der Überwachungs-Konnektor 13 dient also einerseits zum Verbinden mit der Atemluft-Verbindungs-Schlauchkomponente 11, also mit einem beheizbaren Atemluft-Schlauchabschnitt zur Führung von Beatmungsluft von dem Hauptaggregat 2 hin zum Patienten, und andererseits zum Verbinden dieser Schlauchkomponente 11 mit der Patienten-Atemmaske 15 als Patienten-Luftinterface.

Bei einer nicht näher dargestellten Variante ist der Überwachungs-Konnektor 13 mit der Schlauchkomponente 11 integral verbunden, bildet also einen integralen Bestandteil mit dieser.

Über den Dreiwege-Konnektor 14 und einem weiteren Anschluss-Konnektor 16 steht die Atemmaske 15 mit einer Atemluft-Abführ-Schlauchkomponente 17 in Fluidverbindung. Diese steht über einen weiteren Anschluss-Konnektor 18 und einem weiteren Anschlussport 19 wiederum mit dem Hauptaggregat 2 in Fluidverbindung.

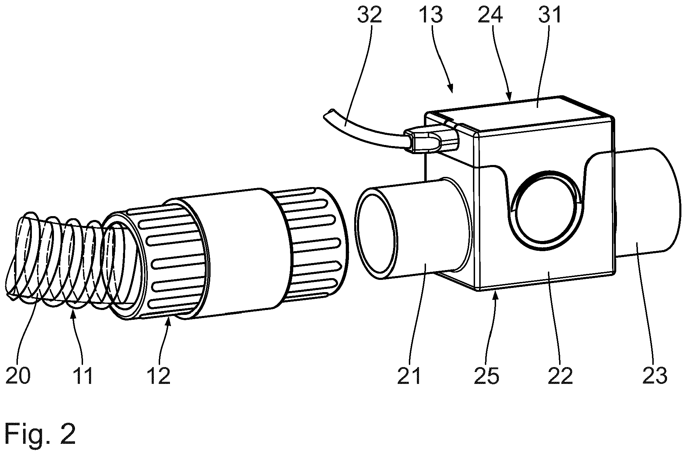

Fig. 2 zeigt einen Abschnitt der Atemluft-Verbindungs-Schlauchkomponente 11 zusammen mit dem Anschluss-Konnektor 12 und, hiervon separat dargestellt, dem Überwachungs-Konnektor 13. Die Schlauchkomponente 11 hat eine äußere Heizwendel 20, ausgeführt als elektrische Widerstandsheizung, zur Beheizung der in der Schlauchkomponente 11 geführten Atemluft. Der Anschluss-Konnektor 12 hat eine in der Fig. 2 nicht sichtbare Aufnahme, die komplementär zu einer Zuführ-Anschlusshülse 21 des Überwachungs-Konnektors 13 ausgeführt ist. Die Zuführ-Anschlusshülse 21 ist an einen Grundkörper 22 des Überwachungs-Konnektors 13 angeformt. Gegenüberliegend zur Zuführ-Anschlusshülse 21 ist an den Grundkörper 22 eine Abführ-Anschlusshülse 23 des Überwachungs-Konnektors 13 angeformt. Ein Außendurchmesser der Zuführ-Anschlusshülse 21 ist kleiner als ein Außendurchmesser der Abführ-Anschlusshülse 23. Über die beiden Anschlusshülsen 21, 23 und eine hierzu fluchtende Öffnung im Grundkörper 22 ist ein Atemluftfluss durch den Überwachungs-Konnektor 13 gewährleistet.

Bei einer nicht dargestellten Ausführung entfällt die Zuführ-Anschlusshülse 21 und auch der Anschlusskonnektor 12 und der Grundkörper 22 des Überwachungs-Konnektors 13 ist direkt an die Schlauchkomponente 11 angeformt, so dass eine Luftführungs-Konnektorkomponente des Überwachungs-Konnektors 13, also der Grundkörper 22, einen integralen Bestandteil mit der Atemluft-Schlauchkomponente 11 bildet.

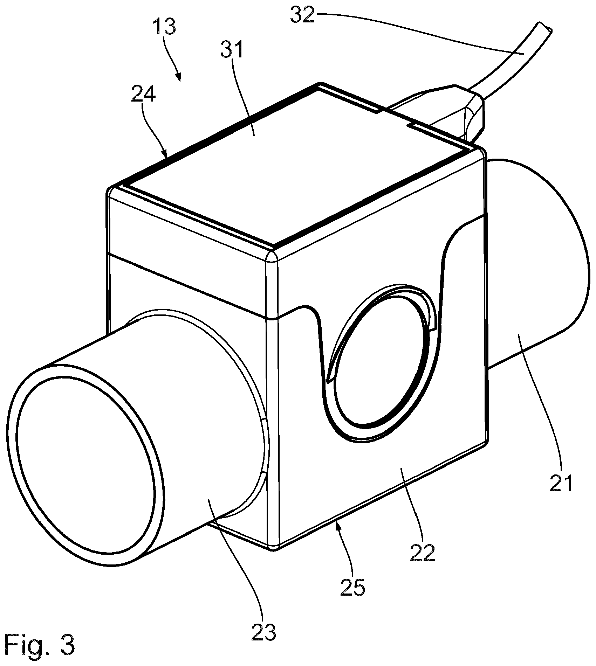

Fig. 3 bis 6 zeigen weitere Details des Überwachungs-Konnektors 13.

Der Überwachungs-Konnektor 13 hat als Hauptbestandteile eine wiederverwendbare Komponente 24, die auch als Reusable bezeichnet wird, und eine Einweg-Komponente 25, die auch als Disposable bezeichnet wird. Grundsätzlich ist es möglich, das Disposable 25 nach entsprechender Reinigung bzw. Sterilisation auch mehrfach zu benutzen.

Das Reusable 24 ist mit dem Disposable 25 lösbar über eine elektrische Steckerverbindung 26 verbunden. Die Steckerverbindung ist mehrpolig und in der dargestellten Ausführung achtpolig ausgeführt. In der dargestellten Ausführung ist ein Stecker 27 der Steckerverbindung 26 als Teil des Disposables 25 ausgeführt.

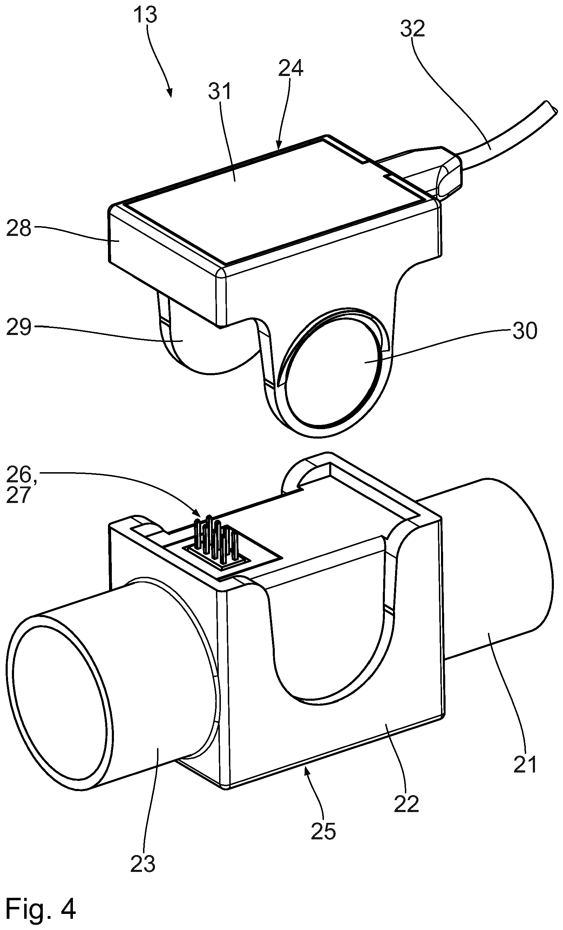

Das Reusable 24 hat einen quaderförmigen Grundkörper 28, von dem aus sich zwei Überstandskomponenten 29, 30 wegerstrecken, die im mit dem Disposable 25 zusammengesetzten Zustand zwei gegenüberliegende Seitenwände des Disposables 25 abschnittsweise überdecken. Bis auf einen den Grundkörper 28 in der Orientierung nach Fig. 4 nach oben hin abschließenden Deckel 31 sind Sichtseiten des Grundkörpers 28 aus transparentem bzw. opakem Material ausgeführt. Der Grundkörper 28 weist zudem eine Versorgungs-Steckverbindung zum Anschluss einer Versorgungsleitung 32 auf, über die eine elektrische Versorgung und/oder eine Signalübertragung von/zu einer externen Komponente, insbesondere dem Hauptaggregat 2 möglich ist.

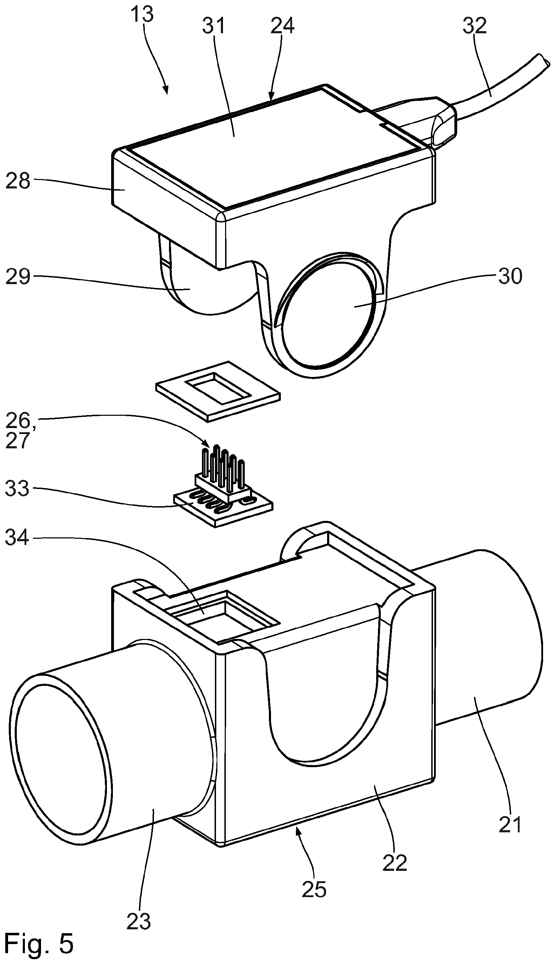

Fig. 5 zeigt in einer zusätzlichen Explosionsdarstellung eine Einbettung einer Platine 33 in einen entsprechenden Aufnahmeraum 34 im Grundkörper 22 des Disposables 25. Der Aufnahmeraum 34 steht über eine Ankopplungswand, über ein Fenster oder über eine Durchtrittsöffnung mit einem die beiden Anschlusshülsen 21, 23 verbindenden Innenlumen des Grundkörpers 22 in Verbindung. Dies kann zu einer sensorischen Ankopplung eines im Aufnahmeraum 34 untergebrachten Sensors des Disposables 25 an die hierüber geführte Atemluft genutzt werden.

Der Überwachungs-Konnektor 13 hat eine Steuer/Regeleinheit 35. Diese ist in einem Aufnahmeraum 36 des Reusables 24 untergebracht. Kernkomponente der Steuer/Regeleinheit 35 ist ein Mikrocontroller 37, der nebst anderen Komponenten auf einer Platine 38 im Aufnahmeraum 36 untergebracht ist. Zur Steuer/Regeleinheit 35 gehört weiterhin eine Echtzeituhr (Real Time Clock) 39, die einen Quarzkristall-Zeitgeber aufweisen kann, und ein Verbindungscontroller 40, der ebenfalls als Mikrocontroller ausgeführt ist und eine Datenverbindung zwischen den Bauteilen auf der Platine 38 des Reusables 24 und den Bausteinen auf der Platine 33 des Disposables 25 steuert. Als zusätzliche Komponente der Steuer/Regeleinheit 35 kann ein Sensorcontroller 41 auf der Platine 38 vorgesehen sein. Der Mikrocontroller 37 und die Echtzeituhr 39 sind auf einer Seite der Platine 38 und der Verbindungscontroller 40 und der Sensorcontroller 41 auf der gegenüberliegenden Seite der Platine 38 untergebracht, auf der auch eine Buchse der Steckerverbindung 26 angeordnet ist.

Die auf den Platinen 33 bzw. 38 angeordneten elektronischen Bauteile können als SMD-Komponenten ausgeführt sein. Bei den Platinen 33, 38 kann es sich um zweilagige PCBs handeln. Eine interne Kommunikation zwischen den Bauelementen auf den Platinen 33, 38 kann über einen I²C-Schnittstellenstandard erfolgen.

Auf der Platine 38 sind weiterhin zwei Lichtquellen 42 angeordnet, die als RGB LEDs ausgeführt sind und mit der Steuer/Regeleinheit 35 in Signalverbindung stehen. Je nach Ansteuerung über den Mikrocontroller 37 kann die jeweilige Lichtquelle 42 also beispielsweise rotes Licht, grünes Licht, blaues Licht oder auch weißes Licht emittieren. Dieses emittierte Licht ist durch die transparenten/opaken Abschnitte des Grundkörpers 28 des Reusable 24 sichtbar.

Die Anzahl der Lichtquellen 42 kann je nach Ausführung des Überwachungs-Konnektors 13 zwischen 1 und 10 variieren.

Die jeweilige Lichtquelle 42 hat über diese RGB LEDs also eine Mehrzahl von Einzel-Lichtquellen verschiedener Farben. Gesteuert über den Mikrocontroller 37 ist jede dieser Farben einem Zustand des Überwachungs-Konnektors 13 bzw. einem Zustand des Beatmungssystems 1 zugeordnet. Zudem kann der Mikrocontroller 37 eine Aktvierungsfrequenz der Lichtquelle 42 vorgeben, so dass beispielsweise weitere Zustände des Überwachungs-Konnektors 13 bzw. des Beatmungssystems 1 über eine Blinksequenz der Lichtquelle 42 angezeigt werden können.

Über eine Lichtführung des von den Lichtquellen 42 emittierten Lichts über die transparenten/opaken Abschnitte des Grundkörpers 28 sowie die Überstands-Komponenten 29, 30 ist gewährleistet, dass das von den Lichtquellen 42 generierte Lichtsignal aus mindestens fünf Raumrichtungen sichtbar ist. Eine Sichtbarkeit direkt von oben, also aus einer Blickrichtung senkrecht zur Anordnungsebene des Deckels 31, ist dadurch geleistet, dass der transparente/opake Grundkörper 28 vollumfänglich um den Deckel 31 herum überstehend ausgeführt ist. Die anderen vier Raumrichtungen, aus denen ein Lichtsignal der Lichtquellen 42 sichtbar sind, sind die den vier Seitenwänden des Grundkörpers 28 zugeordneten Hauptrichtungen.

Auf der Platine 33 des Disposables 25 ist zudem ein Speicherbaustein 43 angeordnet. Als ROM-Daten beinhaltet der Speicherbaustein einen Identifikations-Datensatz, welcher das Disposable 25 eindeutig identifiziert. Bei dem Identifizierungssignal kann es sich beispielsweise um eine individuelle Kennnummer des Disposables 25 handeln, die bei der Herstellung des Disposables 25 vergeben wird.

Zusammen mit dem Mikrocontroller 37 und ggf. dem Verbindungscontroller 40 stellt der Speicherbaustein 43 eine Startsignal-Generatoreinheit 44 zur Erzeugung eines Startsignals dar, ab dem die Steuer/Regeleinheit 35 eine Nutzungsdauer des Disposables 25 verfasst. Hierzu erzeugt eine Übertragung des Identifikations-Datensatzes zwischen dem Speicherbaustein 43 und dem Mikrocontroller 37 das Startsignal. Alternativ oder zusätzlich kann die Startsignal-Generatoreinheit lediglich den Mikrocontroller 37 und ggf. den Verbindungscontroller 40 aufweisen und so ausgeführt sein, dass eine Herstellung eines elektrischen Kontaktes zwischen dem Reusable 24 und dem Disposable 25 über die Steckerverbindung 26 das Startsignal erzeugt. Auch ein Trigger durch das Hauptaggregat 2 kann über eine entsprechende Steuer-Signalverbindung das Startsignal erzeugen.

Die Platine 33 des Disposables 25 trägt weiterhin einen Sensor 45 zur Erfassung eines Atemluft-Parameters. Der Sensor 45 steht über die Steckerverbindung 26 mit dem Mikrocontroller 37 der Steuer/Regeleinheit 35 in Signalverbindung. Bei dem Sensor 45 handelt es sich um einen Temperatursensor. Dieser kann in einem Temperaturbereich zwischen -20°C und 90°C funktionsfähig sein und beispielsweise in einem Bereich zwischen -10°C und 80°C eine Messgenauigkeit von 0,2 K haben. Signaldaten des Sensors 45 können im Speicherbaustein 43 des Disposables 25 und/oder in einem Speicher des Mikrocontrollers 37 zumindest temporär gespeichert werden. Der Mikrocontroller 37 und der Speicherbaustein 43 können somit die Funktion eines Signaldaten-Speichers haben.

Der Mikrocontroller 37 beinhaltet ein Verarbeitungsmodul 37a zur Verarbeitung der Signaldaten.

Der Signaldaten-Speicher des Mikrocontrollers 37 kann seinerseits einen Identifikations-Datensatz als ROM-Datensatz aufweisen, über den das Reusable 24 eindeutig identifiziert werden kann.

Der Sensor 45 steht über ein Koppelmedium 46 und einen dünnen Sensor-Wandabschnitt 47 mit dem Innenlumen 48 des Disposables 25 in sensorischer Verbindung, insbesondere in thermischem Kontakt. Bei dem Koppelmedium 46 handelt es sich entsprechend um sehr gut wärmeleitendes Material.

Eine Wandstärke des Sensor-Wandabschnitts 47 kann geringer sein als 1 mm, kann geringer sein als 0,5 mm, geringer als 0,25 mm und kann auch geringer sein als 0,2 mm. Im Regelfall ist die Wandstärke des Sensor-Wandabschnitts 47 größer als 25 µm.

Der Sensor 45 ist so ausgeführt, dass er den Atemluft-Parameter, also die Atemluft-Temperatur, kontaktlos, also ohne direkten Kontakt mit der Atemluft, misst.

Das Koppelmedium 46 einerseits und der Sensor-Wandabschnitt 47 andererseits stellen eine Decklage dar, über die der Sensor 45 hin zum Lumen 48 des Disposables 25, also hin zu einem Atemluft-Führungslumen, abgedeckt ist.

Alternativ zu einem Koppelmedium und/oder einem Wandschnitt kann als Decklage 46 auch ein Fenster in den Grundkörper 22 des Disposables 25 eingesetzt sein, über das der Sensor 45 mit dem Lumen 48 und damit mit der Atemluft im sensorischen Kontakt steht.

Bei dem Sensor 45 kann es sich dann um einen optischen Sensor und insbesondere um einen ortsauflösenden optischen Sensor handeln.

Bei einem alternativ oder zusätzlich möglichen Sensor 45a, der ebenfalls von der Platine 33 getragen wird, misst der Sensor 45a den jeweiligen Atemluft-Parameter in Kontakt mit der Atemluft. Hierzu ist anstelle des Sensor-Wandabschnitts 47 im Grundkörper 22 des Disposables 25 ein Sensorfenster bzw. eine Sensorausnehmung 22a ausgeführt, über welches eine Sensorfläche des Sensors 45a direkt mit der Atemluft im Lumen 48 in Kontakt kommt. Die sensitive Sensorfläche des Sensors 45a ist so angeordnet, dass zwischen dieser und dem Lumen 48 kein störendes Totvolumen vorliegt. Bevorzugt schließt die sensitive Sensorfläche des Sensors 45a bündig mit einer Kanalwandung des Sensorfensters bzw. der Sensorausnehmung 22a im Lumen 48 ab.

Bei einem derartigen Sensor 45a, der den Atemluft-Parameter im direkten Kontakt mit der Atemluft misst, kann es sich beispielsweise um einen Analysesensor zur Bestimmung einer Zusammensetzung des Atemgases, um einen Sensor zur Messung einer Durchflussmenge der Atemluft oder um einen Feuchtesensor für die Atemluft handeln.

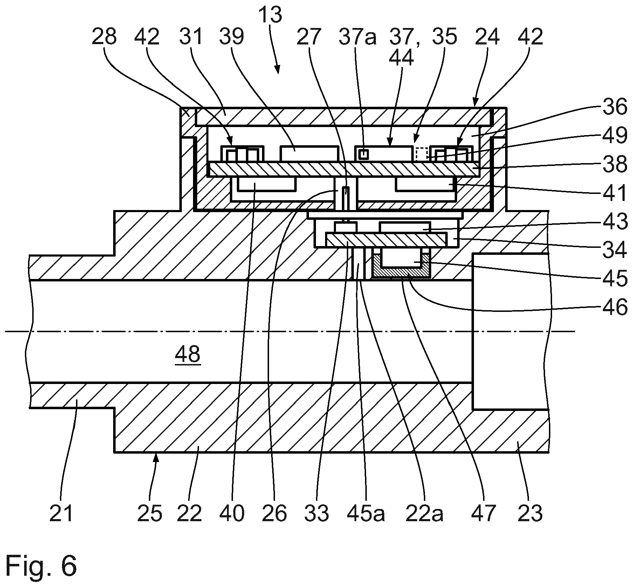

Der Überwachungs-Konnektor 13 kann zusätzlich einen Umgebungssensor zur Erfassung eines Umgebungsparameters aufweisen, der mit der Steuer/Regeleinheit 35 in Signalverbindung steht. Ein derartiger Sensor kann beispielsweise ebenfalls auf der Platine 38 angeordnet sein, was in der Fig. 6 bei 49 angedeutet ist. Der Umgebungssensor kann, ähnlich wie dies vorstehend im Zusammenhang mit dem Sensor 45 beschrieben wurde, über einen dünnen Sensor-Wandabschnitt des Grundkörpers 28 oder über ein hierin ausgebildetes Fenster mit der Umgebung in Sensorkontakt stehen. Der Umgebungssensor 49 kann ein Mikrofon aufweisen. Der Umgebungssensor 49 kann als Bewegungserkennungssensor zum Erkennen einer Bewegung des Überwachungs-Konnektors 13 ausgeführt sein, beispielsweise als Beschleunigungssensor.

Als Schnittstellen-Standards können RS232 und/oder RS485 zum Einsatz kommen.

Über die Steckerverbindung 26 sowie den Verbindungscontroller 40 sind mehrere Signalübertragungs-Schnittstellen der Steuer/Regeleinheit 35 realisiert. Es ist also eine Signalübertragung nach mehreren und insbesondere verschiedenen Schnittstellen-Standards möglich. Eine interne Kommunikation zwischen den Bauelementen auf den Platinen 33, 38 kann über einen I²C-Schnittstellenstandard erfolgen. Mindestens eine dieser Schnittstellen ist zur Signalverbindung mit dem Sensor 45 ausgeführt und entsprechend mit diesem verbunden. Eine weitere dieser Schnittstellen ist zur Signalverbindung mit einem noch nicht im diesem Überwachungs-Konnektor 13 verbauten Sensor vorbereitet. Auf diese Weise lässt sich eine Erweiterbarkeit des Überwachungs-Konnektors um zusätzliche Sensoren gewährleisten, die mit anderen Schnittstellen-Standards als der Sensor 45 arbeiten.

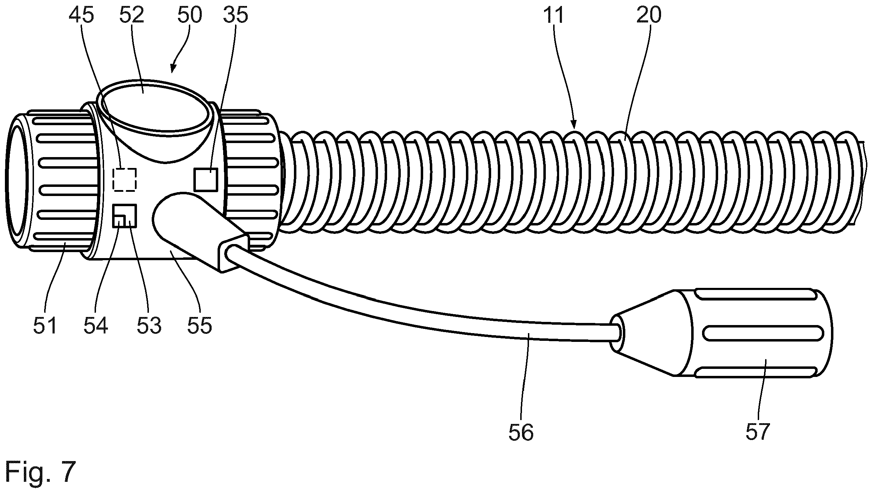

Anhand der Fig. 7 wird nachfolgend eine weitere Ausführung eines Überwachungs-Konnektors 50 für das Patienten-Beatmungssystem erläutert.

Komponenten und Funktionen, die denjenigen entsprechen, die vorstehend unter Bezugnahme auf die Fig. 1 bis 6 bereits erläutert wurden, tragen die gleichen Bezugsziffern und werden nicht nochmals im Einzelnen diskutiert.

Der Überwachungs-Konnektor 50 integriert die Funktionen des Anschluss-Konnektors 12 und des Überwachungs-Konnektors 13 der Ausführung nach Fig. 2. Der Überwachungs-Konnektor 50 dient also einerseits zur mechanischen und atemluftführenden Konnektierung der Atemluft-Verbindungs-Schlauchkomponente 11 an den Dreiwege-Konnektor 14 und hat andererseits die vorstehend erläuterten Steuer/Regel- sowie Überwachungsfunktionen des Überwachungs-Konnektors 13. Der Überwachungs-Konnektor 50 ist als Konnektor ausgeführt, der die Schlauchkomponente 11 mit der Patienten-Atemmaske 15, also dem Patienteninterface, verbindet. Weiterhin hat der Überwachungs-Konnektor 50 mindestens einen Grundsensor nach Art des Sensors 45 zur Erfassung eines Atemluft-Messparameters. Der Grundsensor 45 ist in der Fig. 7 nochmals gestrichelt angedeutet. Der Überwachungs-Konnektor 50 ist mit der Schlauchkomponente 11 integral verbunden und insbesondere mit dieser vergossen.

Eine Luftführungs-Konnektorkomponente des Überwachungs-Konnektors 50, also der das Innenlumen 48 vorgebende Grundkörper 51, bildet einen integralen Bestandteil mit einem Innenlumen der Schlauchkomponente 11. Der Grundkörper 51 des Überwachungs-Konnektors 50 kann den Sensor nach Art des Sensors 45 aufweisen. Eine Elektronik-Konnektorkomponente 52 des Überwachungs-Konnektors 50, die die Steuer/Regeleinheit 35 aufweist, ist lösbar mit dem Grundkörper 51 des Überwachungs-Konnektors 50 verbunden, beispielsweise über eine mechanische Steckverbindung. Zudem kann der Überwachungs-Konnektor 50 eine zusätzliche Sensor-Konnektorkomponente 53 aufweisen, die einen Erweiterungssensor 54 zur Erfassung eines weiteren Atemluft-Parameters, also eines anderen Parameters als durch den Grundsensor erfasst.

Bestandteil der Elektronik-Konnektor-Komponente 52 kann ein RFID-Chip, insbesondere zur Übertragung eines Identifikations-Datensatzes, sein.

Über einen Verbindungsabschnitt 55 ist eine Versorgungsleitung 56 nach Art der Versorgungsleitung 32 mit dem Überwachungs-Konnektor 50 verbunden. Über einen Versorgungsstecker 57 kann die Versorgungsleitung 56 an ein nicht dargestelltes Versorgungsgerät zur Energie-/Signalübertragung angeschlossen werden, beispielsweise an das Hauptaggregat 2 des Beatmungssystems 1.

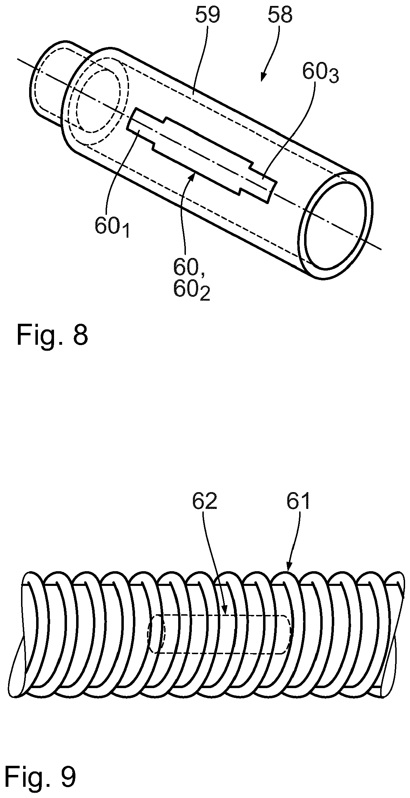

Anhand der Fig. 8 wird nachfolgend eine weitere Ausführung eines Überwachungs-Konnektors 58 beschrieben. Komponenten und Funktionen, die denjenigen entsprechen, die vorstehend unter Bezugnahme auf die Fig. 1 bis 7 bereits erläutert wurden, tragen die gleichen Bezugszeichen und werden nicht nochmals im Einzelnen diskutiert.

Dargestellt ist in der Fig. 8 ein Disposable des Überwachungs-Konnektors 58 nach Art des Disposables 25. Alternativ kann es sich beim Überwachungs-Konnektor 58 um einen integrierten Konnektor nach Art des Überwachungs-Konnektors 50 handeln.

Ein Abschnitt 60 eines Grundkörpers 59 des Überwachungs-Konnektors 58, der gleichzeitig einen Wandabschnitt des Innenlumens 48 bildet, ist aus einem thermochromen Material ausgeführt. Eine Farbumschlagssensitivität des Abschnitts 60 ist an einen vorgegebenen Betriebstemperatur-Bereich der zu führenden Atemluft angepasst.

Der Abschnitt 60 ist erfindungsgemäß in mehrere Abschnitte 60₁, 60₂, 60₃ unterteilt.

Diese Abschnitte 60₁, 60₂, 60₃ sind erfindungsgemäß jeweils aus thermochromen Materialien mit unterschiedlichen Farbumschlags-Temperaturen ausgeführt. Erfindungsgemäß ist mindestens einer dieser Abschnitte 60₁, 60₂, 60₃ aus einem reversiblen thermochromen Material und mindestens ein weiterer dieser Abschnitte 60₁, 60₂, 60₃ aus einem nicht reversiblen thermochromen Material ausgeführt.

Anhand der Fig. 9 wird nachfolgend eine weitere Ausführung einer Schlauchkomponente nach Art der Schlauchkomponenten 5, 11, 17 beschrieben, die vorstehend insbesondere unter Bezugnahme auf die Fig. 1 bis 7 bereits erläutert wurden. Komponenten und Funktionen, die denjenigen entsprechen, die vorstehend unter Bezugnahme insbesondere auf die Fig. 1 bis 7 bereits erläutert wurden, tragen die gleichen Bezugsziffern und werden nicht nochmals im Einzelnen diskutiert.

Bei der Schlauchkomponente 61 nach Fig. 9 ist ein Teil einer Schlauchwand als Abschnitt 62 aus thermochromen Material ausgeführt. Für die insbesondere abschnittsweise Gestaltung des Abschnitts 62 der Schlauchkomponente 61 gilt, was vorstehend zu den Abschnitten 60ᵢ des Abschnitts 60 nach Fig. 8 ausgeführt wurde.

Ein Grundbestandteil des thermochromen Materials kann ein thermoplastisches Elastomer sein oder Silikon.

Auch bei den vorstehend bereits beschriebenen Konnektoren 13 bzw. 50 kann eine Komponente bzw. ein Abschnitt einer Komponente, die thermisch an die geführte Atemluft angekoppelt ist, aus thermochromen Material gefertigt sein.

Bei dem thermochromen Material kann es sich um eine Materialzusammensetzung enthaltend anorganische Verbindungen von Rutil und Zinkoxid handeln. Alternativ oder zusätzlich können Bestandteile mit Bixanthyliden-Derivaten und/oder Bianthronyliden-Derivaten zum Einsatz kommen. Grundsätzlich können auch Silberjodid-Verbindungen zum Einsatz kommen. Alternativ oder zusätzlich kann auch Bromthymolblau, eingebettet in eine ph-abhängige Polymermatrix, zum Einsatz kommen. Weitere Varianten, die Bestandteile des thermochromen Materials sein können, sind eine lithiumchloridhaltige Polyethermatrix, Bis(diethylammonium)-tetrachloridocuprat(II) und salvatochrome Farbstoffe.

Das Beatmungssystem 1 kann folgendermaßen genutzt werden:
In einem Bereitstellungzustand liegt der Überwachungs-Konnektor 13 mit vom Dispoable 25 jeweils getrenntem Reusable 24 vor. Wenn die Schlauchkomponenten 5, 11 und 17 miteinander und mit dem Hauptaggregat 2 verbunden werden, um das Beatmungssystem 1 einsatzfähig zu machen, wird kurz vor Beginn des ersten Einsatzes das Reusable 24 mit dem Disposable 25 verbunden, wobei das Startsignal durch die Startsignal-Generatoreinheit 37 bzw. 37, 43 erzeugt wird, wie vorstehend erläutert. Ab dem dann festgelegten Start-Zeitpunkt erfasst der Mikrocontroller 37 in Signalverbindung mit der Echtzeituhr 39 den erfassten Zeitraum und überwacht somit eine Einsatzdauer des Disposables 25 bei der Beatmung. Ausgehend von einer maximalen Einsatzdauer von sieben Tagen kann die Steuer/Regeleinheit 35 die Lichtquelle 42 beispielsweise so ansteuern, dass während der ersten sechseinhalb Tage die Lichtquellen 42 grünes Licht emittieren. Die verbleibenden 12 Stunden bis zum Erreichen der maximalen Einsatzdauer kann das Licht dann von grün auf blau umschalten, was von der Steuer/Regeleinheit 35 gesteuert wird. Bei Überschreitung der sieben Tages-Einsatzdauer kann dann zunächst ein blaues Blinksignal erzeugt werden, welches nach Ablauf einer Karenzzeit in ein rotes Signal, jeweils wiederum gesteuert über die Steuer/Regeleinheit 35, übergeht. Es werden jedenfalls bei Erreichen der Maximal-Einsatzdauer die mit der Atemluft in Berührung kommenden Komponenten des Beatmungssystems 1 getauscht. Das Reusable 24 wird dann wiederverwendet und mit dem neuen Disposable 25 der getauschten Atemluft-Führungskomponenten verbunden, so dass der Einsatzdauer-Zyklus nach Erzeugung des Startsignals durch die Startsignal-Generatoreinheit 37 bzw. 37, 43 neu beginnen kann.

Der Überwachungs-Konnektor 13 kann zudem zur Temperaturmessung und insbesondere zu einer Temperaturschwellwerterkennung heranzogen werden. Hierzu wird die Atemlufttemperatur über den Sensor 45 erfasst und ein entsprechendes Temperatursignal an den Mikrocontroller 37 weitergegeben. Sobald die gemessene Temperatur einen Schwellwert, beispielsweise 40°C überschreitet, steuert die Steuer/Regeleinheit 35 die Lichtquellen 42 zur Ausgabe eines visuellen Warnsignals, beispielsweise eines roten Blinklichts, an. Sobald nach Überschreiten der Temperaturschwelle ein zweiter, definiert niedrigerer Temperaturwert von der gemessenen Atemluft-Temperatur unterschritten wird, kann diese visuelle Anzeige wieder, gesteuert über die Steuer/Regeleinheit 35, beendet werden.

Die Speichereinheiten als Teil des Mikrocontrollers 37 bzw. 43 können zudem zum Speichern einer digitalen Visitenkarte des Überwachungs-Konnektors 13 insgesamt oder auch des Reusables 24 bzw. des Disposables 25 eingesetzt werden. Teil eines entsprechenden Visitenkarten-Datensatzes können sein der Identifikations-Datensatz, eine Typenbezeichnung des Überwachungs-Konnektors 13 sowie des gesamten Beatmungssystems 1, eine Gebrauchsanweisung für den Überwachungs-Konnektor und/oder das Beatmungssystem sowie weitere Daten, beispielsweise für relevante Nutzungsdaten. Der Identifizierungs-Datensatz kann als Plagiatsschutz ausgeführt sein. Als Nutzungsdaten im Speicher des Mikrocontrollers 37 können beispielsweise die über die Lebensdauer des Reusables 24 zum Einsatz gekommenen IDs der Disposables 25 gespeichert sein oder auch Messdatenverläufe des Sensors 45 über die Zeit sowie über die Ansteuersequenzen der Lichtsignale 2 mittels der Steuer/Regeleinheit 35 erfasste Konnektorzustände, insbesondere Alarmereignisse (Überschreitung der maximalen Nutzungsdauer/Temperaturschwellwertüberschreitung).

Der Sensor 45 kann mit einem integrierten EEPROM ausgeführt sein. Hierüber können insbesondere Temperaturschwellwerte durch Programmierung vorgegeben werden. In dem Speicher des Sensors 45 kann auch ein eigener Identifikations-Datensatz des Disposables 25 gespeichert sein, der bei der Herstellung des Disposables 25 dort hinterlegt werden kann. Alternativ kann ein solcher Identifikations-Datensatz von dem Mikrocontroller 37 des Reusables 24 bei der elektrischen Verbindung dieser beiden Komponenten oder auch beim Start der Nutzungsdauer in den Speicher des Sensors 45 geschrieben werden.

Über eine drahtlose oder auch drahtgebundene Schnittstelle kann die Steuer/Regeleinheit 35 mit einer externen Anzeigeeinheit in Signalverbindung stehen, beispielsweise mit einem Graphical User Interface (GUI). Hierüber ist beispielsweise eine Anzeige der Momentantemperatur oder eine Anzeige der aktuellen Nutzungsdauer sowie weiterer Sensordaten möglich.

Im Speicherbaustein 43 bzw. im Speicherbaustein des Mikrocontrollers 37 kann ein Logfile geschrieben werden, welches die Zustandsparameter des Überwachungs-Konnektors 13 protokolliert.

Alternativ oder zusätzlich zum Temperatursensor 45 kann ein Feuchtesensor, ein Massenfluss- bzw. Strömungssensor, ein Sensor zur Bestimmung einer Kondenswasser-Agglomeration oder auch ein Gasanalyse-Sensor zur Bestimmung einer Gaszusammensetzung der Atemluft und insbesondere zur Überwachung einer Kontamination und/oder eines Überschreitens kritischer Bestandteilschwellwerte zum Einsatz kommen.

Soweit ein Atemluft-Drucksensor im Überwachungs-Konnektor 13 zum Einsatz kommt, kann dieser zur Regelung einer Atemanstrengungsanpassung genutzt werden.

Zudem kann ein Druck- bzw. Dehnungssensor zum Erfassen einer mechanischen Belastung des Überwachungs-Konnektors 13 zum Einsatz kommen. Ein solcher Sensor kann eine Membranstruktur und/oder eine Mäanderstruktur aufweisen. Eine Druck- bzw. Kraftbeaufschlagung führt bei einem derartigen Druck- bzw. Dehnungssensor zu einer Strukturdehnung, die detektiert und in Druck- und/oder Kraftwerte umgerechnet werden kann. Ein solcher Drucksensor kann in einem Abschnitt des Überwachungs-Konnektors 13, z. B. im Reusable 24 oder im Disposable 25, angeordnet sein. Alternativ oder zusätzlich kann ein solcher Druck- bzw. Dehnungssensor als externer Sensor beispielsweise in der Atemluft-Schlauchkomponente 11 angeordnet sein und mit dem Überwachungs-Konnektor 13 über entsprechende Schnittstellen in Signalverbindung stehen.

Ein derartiger Druck- bzw. Dehnungssensor kann alternativ oder zusätzlich auch eine Druckbelastung der Schlauchkomponenten 5, 11 oder 17 überwachen. Auf diese Weise ist es möglich, eine unerwünschte Belastung einer dieser Komponenten, z. B. eine Knickung oder Biegung der Atemluft-Schlauchkomponente 5, 11 oder 17, des Beatmungssystems 1 zu erfassen, um ggf. durch visuellen oder Audio-Alarm schnell Gegenmaßnahmen zu greifen.

Weitere Sensoren, die im Beatmungssystem 1 entweder integriert in den Überwachungs-Konnektor oder als externe Sensoren genutzt werden können, sind ein Sensor zur Haut-Temperaturmessung des Patienten, ein Sensor zur Hautfeuchtemessung des Patienten, ein Sensor zur Hautfarbenmessung des Patienten, ein Herzfrequenz-Sensor, ein Sauerstoff-Sättigungssensor, ein Gas-Analysesensor, ein Brandschutzmelder, ein Orientierungssensor zur Bestimmung einer Lage mindestens einer Komponente des Beatmungssystems 1 im Raum, ein Bewegungssensor zur Bestimmung einer Bewegung mindestens einer Komponente des Beatmungssystems 1, ein Sensor zur Überwachung einer Arzneimittelabgabe.

In dem Mikrocontroller 37 kann eine Verarbeitung, insbesondere eine Vorverarbeitung der an den Mikrocontroller 37 übertragenen Sensordaten erfolgen. Beispielsweise können die Daten gemittelt oder gefiltert oder zur Verringerung des Speicherbedarfs komprimiert werden. Diese (vor-)verarbeiteten Daten können dann zur externen Weiterverarbeitung bzw. Anzeige beispielsweise an das Hauptaggregat 2 und/oder an eine andere externe Datenverarbeitungskomponente weitergeleitet werden.

Die Atemluft führenden Komponenten des Beatmungssystems 1 können antimikrobiell beschichtet sein.

Über eine Gasflussmessung kann insbesondere durch entsprechende Verarbeitung im Mikrocontroller 37 und/oder im Hauptaggregat 2 auf eine Leckage im Beatmungssystem 1 rückgeschlossen werden.

Der Mikrocontroller 37 kann programmierbar ausgeführt sein. Zudem kann der Speicher des Mikrocontrollers 37 eine Programmbibliothek beinhalten, aus der Programme oder Programmbestandteile ausgewählt werden können.

Das Reusable 24 kann zudem über weitere kabellose Schnittstellen für das Auslesen insbesondere von aufgenommenen Messdaten verfügen, beispielsweise über RFID, über NFC, über Bluetooth oder über WLAN.

Über eine der vorstehend beschriebenen Schnittstellen kann die Steuer/Regeleinheit 35 mit mindestens einem zum Konnektor 13, 50 oder 58 externen Sensor in Signalverbindung stehen. Als Beispiel für einen derartigen Sensor ist in der Fig. 1 schematisch bei 63 ein derartiger Sensor angedeutet, bei dem es sich um einen Sensor zur Messung einer Herzfrequenz des Patienten, um einen Sensor zur Messung einer Körpertemperatur des Patienten und/oder um einen Sensor zur Messung eines Haut-Oberflächenwiderstandes des Patienten handeln kann.

## Patentansprüche

1. Konnektor (58) für ein Patienten-Beatmungssystem
- zum Verbinden mit einem beheizbaren Atemluft-Schlauchabschnitt (11) zur Führung von Beatmungsluft von einer Atemluftquelle (2) hin zu einem Patienten,
- zum Verbinden des Atemluft-Schlauchabschnitts (11) mit einem Patienten-Luftinterface (15),
- wobei mindestens ein Abschnitt (60; 60₁, 60₂, 60₃; 62) des Konnektors (58) aus einem thermochromen Material ausgeführt ist,
- wobei der Konnektor (58) mehrere Abschnitte (60₁, 60₂, 60₃) aufweist, die jeweils aus einem thermochromen Material mit unterschiedlichen Farbumschlags-Temperaturen ausgeführt sind,
- wobei mindestens einer der Abschnitte (60₁, 60₂, 60₃) aus einem reversiblen thermochromen Material und das mindestens ein weiterer der Abschnitte (60₁, 60₂, 60₃) aus einem nicht reversiblen thermochromen Material ausgeführt ist.

2. Konnektor nach Anspruch 1, **gekennzeichnet durch** einen kontinuierlichen Farbverlauf des reversiblen Abschnitts des Konnektors aus thermochromen Material in Abhängigkeit von der Temperatur.

3. Konnektor nach Anspruch 1 oder 2, **gekennzeichnet durch** einen kontinuierlichen Farbverlauf des nicht reversiblen Abschnitts des Konnektors aus thermochromem Material in Abhängigkeit von der Temperatur.

4. Baugruppe mit mindestens einem Konnektor nach einem der Ansprüche 1 bis 3 und mit einer Atemluft-Schlauchkomponente (11) zur Führung der Beatmungsluft.

5. Baugruppe nach Anspruch 4, **dadurch gekennzeichnet, dass** mindestens ein Schlauchabschnitt (62) der Atemluft-Schlauchkomponente mit der Beatmungsluft in Kontakt steht oder thermisch an die Beatmungsluft angekoppelt ist, wobei der Schlauchabschnitt (62) aus einem thermochromen Material ausgeführt ist.

## Claims

1. Connector (58) for a patient ventilation system
- for connection to a heatable breathing air tube section (11) to guide ventilation air from a breathing air source (2) to a patient,
- for connection of the breathing air tube section (11) to a patient air interface (15),
- wherein at least one section (60; 60₁, 60₂, 60₃; 62) of the connector (58) is made of a thermochromic material,
- wherein the connector (58) has several sections (60₁, 60₂, 60₃) which are each made of a thermochromic material with different colour change temperatures,
- wherein at least one of the sections (60₁, 60₂, 60₃) is made of a reversible thermochromic material and at least one other of the sections (60₁, 60₂, 60₃) is made of a non-reversible thermochromic material.

2. Connector according to claim 1, **characterized by** a continuous colour gradient of the reversible section of the connector made of thermochromic material depending on the temperature.

3. Connector according to claim 1 or 2, **characterized by** a continuous colour gradient of the non-reversible section of the connector made of thermochromic material depending on the temperature.

4. Assembly with at least one connector according to one of the claims 1 to 3 and with a breathing air tube component (11) to guide the ventilation air.

5. Assembly according to claim 4, **characterized in that** at least one tube section (62) of the breathing air tube component is in contact with the ventilation air or coupled thermally to the ventilation air, wherein the tube section (62) is made of a thermochromic material.

## Revendications

1. Raccord (58) pour un système de ventilation de patient destiné
- à être relié à une section de tuyau d'air respiratoire chauffable (11) afin d'acheminer de l'air respiratoire d'une source d'air respiratoire (2) jusqu'à un patient,
- à relier la section de tuyau d'air respiratoire (11) à une interface air-patient (15),
- dans lequel au moins une section (60 ; 60₁, 60₂, 60₃ ; 62) du raccord (58) est réalisée dans un matériau thermochrome,
- dans lequel le raccord (58) présente plusieurs sections (60₁, 60₂, 60₃) qui sont chacune réalisées dans un matériau thermochrome avec des températures de changement de couleur différentes,
- dans lequel au moins une des sections (60₁, 60₂, 60₃) est réalisée dans un matériau thermochrome réversible et au moins une autre des sections (60₁, 60₂, 60₃) est réalisée dans un matériau thermochrome non réversible.

2. Le raccord selon la revendication 1, **caractérisé en ce qu'**il présente un dégradé de couleur continu de la section réversible du raccord en matériau thermochrome en fonction de la température.

3. Le raccord selon la revendication 1 ou 2, **caractérisé en ce qu'**il présente un dégradé de couleur continu de la section non réversible du raccord en matériau thermochrome en fonction de la température.

4. Ensemble doté d'au moins un raccord selon l'une quelconque des revendications 1 à 3 et d'un composant de tuyau d'air respiratoire (11) pour l'acheminement d'air respiratoire.

5. L'ensemble selon la revendication 4, **caractérisé en ce qu'**au moins une section de tuyau (62) du composant de tuyau d'air respiratoire est en contact avec l'air respiratoire ou est couplé à l'air respiratoire de manière thermique, sachant que la section de tuyau (62) est réalisée dans un matériau thermochrome.
